Europäisches Patentamt

European Patent Office

Office européen des brevets

(19)

(11) Veröffentlichungsnummer: **0 006 623**
**B1**

(12) **EUROPÄISCHE PATENTSCHRIFT**

(45) Veröffentlichungstag der Patentschrift: **22.06.83**

(21) Anmeldenummer· **79102163.7**

(22) Anmeldetag: **28.06.79**

(51) Int. Cl.³: **G 10 K 11/34, H 04 R 1/10**

(54) **Ultraschallkopf.**

(30) Priorität: **05.07.78 DE 2829570**

(43) Veröffentlichungstag der Anmeldung:
**09.01.80 Patentblatt 80/1**

(45) Bekanntmachung des Hinweises auf die Patenterteilung:
**22.06.83 Patentblatt 83/25**

(84) Benannte Vertragsstaaten:
**AT CH FR GB NL**

(56) Entgegenhaltungen:
**DE - A - 2 521 463**
**DE - B - 2 829 581**
**DE - C - 732 890**
**US - A - 2 589 135**

(73) Patentinhaber: **SIEMENS AKTIENGESELLSCHAFT**
**Berlin und München Wittelsbacherplatz 2**
**D-8000 München 2 (DE)**

(72) Erfinder: **Borburgh, Jacobus, Dr. Dipl.-Ing.**
**Eichenstrasse 10**
**D-8521 Poxdorf (DE)**
Erfinder: **Feigt, Ingmar, Dr. Dipl.-Phys.**
**Strümpellstrasse 22**
**D-8520 Erlangen (DE)**

Courier Press, Leamington Spa, England.

Die Erfindung bezieht sich auf einen Ultraschallkopf gemäß den Merkmalen des Oberbegriffs des Patentanspruchs 1.

Der Ultraschallkopf kann ein beliebig geformter Abtastkopf für B-Scan oder aber auch für A-Scan oder für ein ähnliches Abtastverfahren sein. Es kann sich im vorliegenden Fall also auch um einen Ultraschallkopf für z.B. Compound-Scan handeln. In besonderer Anwendung sollte der Schallkopf jedoch der Wandlerkamm eines Ultraschall-Arrays sein.

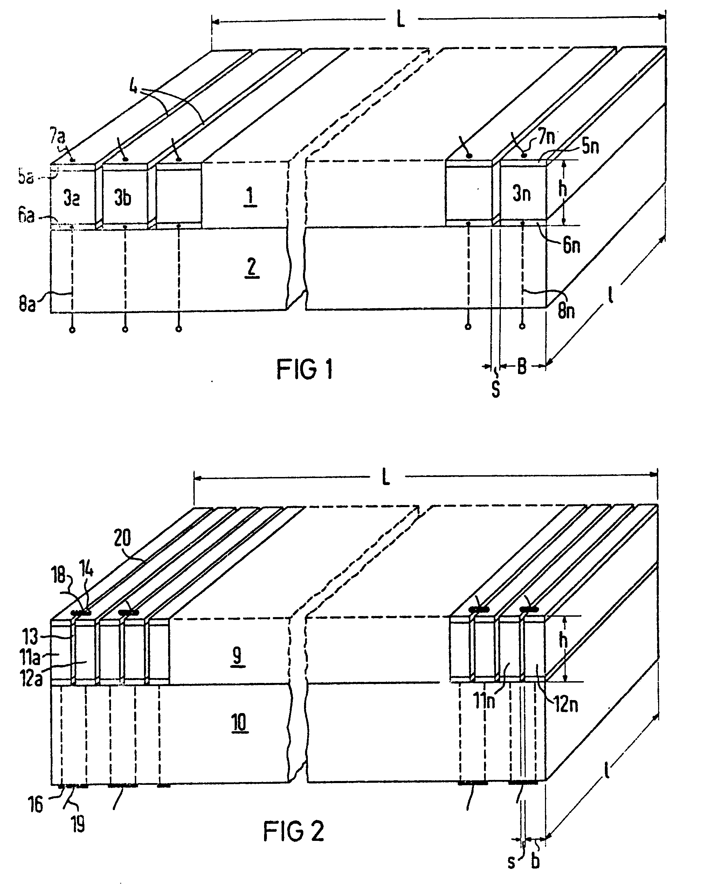

Durch die US—PS 25 89 135 ist bereits ein Ultraschallkopf der eingangs genannten Art vorbekannt, der in Verbindung mit Sonarsystemen in Unterseebooten im Meereswasser eingesetzt wird. Bei diesem Ultraschallkopf beträgt die Höhe jedes einzelnen Wandlerelementes $h = \lambda/4$ (mit $\lambda =$ Wellenlänge der abgestrahlten bzw. empfangenen Ultraschallwellen im Meereswasser). Die Breite b der einzelnen Wandlerelemente liegt nur geringfügig unter $\lambda/4$. Diese Größenverhältnisse zeigt zumindest die Querschnittsdarstellung der Figur 1 der US—PS. Ein solches Verhältnis zwischen Breite und Höhe, die sich in ihren Dimensionen nur geringfügig voneinander unterscheiden, führt zu einer relativ schlechten lateralen Auflösung.

Dies gilt in diesem Sinne auch für einen Wandlerkamm, wie er beispielsweise durch den Artikel "Electronic sector scanning for ultrasonic diagnosis" von J. C. Somer aus der Zeitschrift ULTRASONICS, Juli 1968, Seiten 153 bis 159 bekannt ist. Im speziellen handelt es sich hierbei um ein sogenanntes phased-Array für Sektorabtastung. Die Breite jedes einzelnen Wandlerelementes ist geringfügig kleiner als die Hälfte der Wellenlänge der verwendeten Ultraschallschwingungen. Die Höhe beträgt etwa das 2,6 fache der Wandlerelementbreite. Wie die Praxis zeigt, lassen Wandlerelemente, die etwa $\lambda/2$ breit sind, hinsichtlich der Lateralauflösung immer noch zu wünschen übrig.

Einen Vorschlag, wie bei Wandlerkämmen die Lateralauflösung verbessert werden kann, unterbreitet die nicht vorveröffentlichte US—PS 41 01 795. Gemäß deren Lehre ergibt sich eine verbesserte Lateralauflösung, wenn das Breite-Höhen-Verhältnis im Bereich von 0,4 bis 0,7 liegt. Wird z.B. für die Höhe des Wandlerelementes $h = \lambda/2$ gewählt, so könnte nach der Lehre der US—PS 41 01 795 die Wandlerelementbreite zu $b =$ etwa $\lambda/4$ oder geringfügig kleiner als $\lambda/4$ gewählt werden. Bei einer derartigen Dimensionierung eines einzelnen Wandlerelementes sinkt jedoch die Abstrahl- bzw. Empfangsleistung mit zunehmender Feinteilung der Einzelelemente. D.h., wird ein Ultraschallkopf nach der Lehre der US—PS 41 01 795 mehr und mehr feingeteilt, so verbessert sich zwar die Lateralauflösung; gleichzeitig sinkt aber auch sehr rapide mit abnehmender Abstrahlfläche die Abstrahl- bzw.

Empfangsleistung. Diesem nicht unerheblichen Nachteil versucht der Gegenstand der US—PS 41 01 795 dadurch entgegenzuwirken, daß der gesamte feingeteilte Wandlerkopf applikationsseitig mit einer besonderen doppelbödigen Anpassungsschicht versehen wird, die in der Zusammensetzung recht kompliziert aufgebaut ist.

Aufgabe vorliegender Erfindung ist es, einen Ultraschallkopf der eingangs genannten Art aufzubauen, der in der Lateralauflösung erheblich verbessert ist und der dennoch gleichzeitig mit optimaler Abstrahl- bzw. Empfangsleistung für alle derzeit üblichen Abtastverfahren, also z.B. Compound-Scan, Linear-Array-Scan oder auch als phased-Array für Sektorscan, einsetzbar ist.

Die Aufgabe wird erfindungsgemäß mit den Merkmalen des Kennzeichens des Patentanspruchs 1 gelöst.

Eine entsprechend der Erfindung fein unterteilte Wandlerelementanordnung gewährleistet in der Gruppenzusammenschaltung trotz relativ hohen Teilungsverlustes (Zwischenräume machen beispielsweise 20 % der aktiven Fläche aus) mindestens dieselbe Abstrahl- bzw. Empfangsleistung wie herkömmliche Wandlerelementanordnungen, wobei sich jedoch immer praktisch querschwingungsfreie Echoantwort ergibt. Die Lateralauflösung ist damit bei optimaler Abstrahl- bzw. Empfangsleistung ebenfalls optimal.

Weitere Einzelheiten und Vorteile der Erfindung ergeben sich aus der nachfolgenden Beschreibung eines Ausführungsbeispiels anhand der Zeichnung in Verbindung mit den Unteransprüchen.

Es zeigen:

Figur 1 ein Ultraschall-Array mit herkömmlichem Wandlerkamm;

Figur 2 ein Ultraschall-Array mit Wandlerkamm in Feinunterteilung gemäß der Erfindung;

Figur 3 eine Unterteilungsform in trapezförmigen Schrägschritten über die Länge eines Wandlerstreifens;

Figur 4 ein Einzelsegment der Unterteilung gemäß Fig. 3 in Vergrößerung;

Figur 5 eine Unterteilungsform in trapezförmigen Schrägschritten über die Höhe des Wandlerelementstreifens;

Figur 6 einen gemäß der Erfindung feinunterteilten Ultraschallkopf für Compound-Scan.

Das Ultraschall-Array der Fig. 1 kann vom Prinzip her jenem des eingangs aufgeführten Somer-Artikels entsprechen. Es beinhaltet dann einen Wandlerkamm 1 mit einem Trägerteil 2, z.B. aus Epoxydharz. Der Wandlerkamm umfaßt eine Vielzahl von Wandlerelementen 3a bis 3n aus piezoelektrischem Material (z.B. aus Blei-Zirkonat-Titanat). Die einzelnen Wandlerelemente 3a bis 3n sind auf Abstand S nebeneinander am Trägerteil 2 angeordnet. Gemäß Somer-Artikel sind die Lücken 4 zwischen den einzelnen Wandlerelementen mit Abstandsstücken aus Epoxydharz ausgefüllt. Sie können selbstverständlich (z.B. gemäß Fig. 1) aber auch als Lücken unausgefüllt bleiben. Jedes einzelne Wandlerelement 3a bis 3n weist eine ihm zugeordnete Kontaktierung 5a bis 5n an der Oberseite und 6a bis 6n an der Unterseite (Silberbelege) für den Anschluß von Ansteuerleitungen 7a bis 7n bzw. 8a bis 8n auf. Die Anzahl der Wandlerelemente liegt z.B. im Bereich 125. Die Gesamtlänge des Ultraschall-Arrays ist mit L (ca. 10 cm) bezeichnet. Die Länge der einzelnen Wandlerelemente 3a bis 3n beträgt 1, ihre Höhe ist mit h angegeben und die Breite beträgt B. Die Spaltbreite der Lücken 4 besitzt den Wert S. Die Wandlerelementbreite B liegt im Bereich der Hälfte ($\lambda/2$) der Wellenlänge der verwendeten Ultraschallschwingungen oder allenfalls geringfügig darunter. Die Höhe h ist gemäß den eingangsseitigen Voraussetzungen größer als die Breite B jedes einzelnen Wandlerelementes 3a bis 3n.

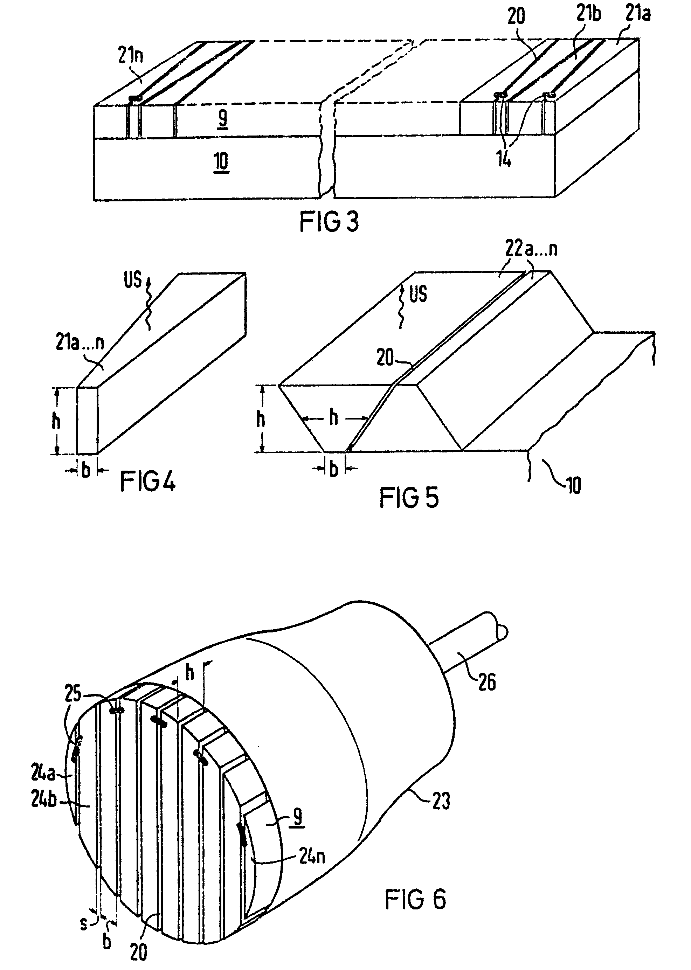

Wie bereits erwähnt, ist die Lateralauflösung eines derartigen Ultraschall-Arrays, wie es z.B. in der Fig. 1 dargestellt ist, noch nicht optimal. Sie wird optimal bei mindestens gleicher Abstrahl- bzw. Empfangsleistung mit dem Ausführungsbeispiel der Fig. 2 (bzw. auch den Ausführungsbeispielen der Fig. 3 bis 6).

Das Ultraschall-Array der Fig. 2 umfaßt wiederum einen Wandlerkamm 9, der an einem Träger 10 (z.B. wieder Epoxydharz) gehaltert ist. Im Gegensatz zum Wandlerkamm 1 des Arrays der Fig. 1 ist jedoch der Wandlerkamm 9 des Arrays der Fig. 2 feinunterteilt. Im dargestellten Ausführungsbeispiel der Fig. 2 befinden sich also anstelle eines einzigen Wandlerelementes 3a bis 3n gemäß der Fig. 1 nun zwei Einzelwandlerelemente 11a, 12a bis 11n, 12n. Die beiden Einzelelemente 11a, 12a bis 11n, 12n weisen jedes eine Breite b auf, die weniger als die Hälfte der Breite B eines Wandlerelementes 3a bis 3n des Arrays der Fig. 1 besitzt. Die Wandlerelementbreite b der Wandlerelemente 11a, 12a und 11n bis 12n in der Feinunterteilung des Ausführungsbeispieles der Fig. 2 ist also kleiner als $\lambda/4$ und dementsprechend auch wesentlich kleiner als die Hälfte ($\lambda/2$) der Wellenlänge der verwendeten Ultraschallschwingungen. Von den Elementen 11a, 12a bis 11n, 12n der Feinunterteilung sind jedoch jeweils immer zwei benachbarte zu einer Gruppe 13 zusammengefaßt. Die gruppenweise Zusammenfassung erfolgt durch Lötstellen 14 an der Oberseite (Abstrahlfläche) und 16 an der Unterseite. Jede Gruppenkontaktierung weist einen eigenen Steuerleitungsanschluß 18 an der Oberfläche und 19 an der Unterseite auf. Innerhalb einer solchen Gruppe 13 ist dann die Summen-Abstrahl-/Empfangsfläche, die von jeweils zwei Wandlerelementen 11a, 12a bis 11n, 12n gemeinsam gebildet wird, einschließlich des vom Spalt 20 gebildeten Zwischenraumes etwa gleich groß der Aktivfläche eines einzelnen Wandlerelementes 3a bis 3n des Ultraschall-Arrays der Fig. 1. Trotz des relativ hohen Spaltverlustes (der Spaltverlust macht ca. 20 % der Aktivfläche aus) ergibt sich für jede der einzelnen Gruppen 13 des Wandlerkammes 9 mindestens dieselbe Abstrahl- bzw. Empfangsleistung wie für jedes der Wandlerelemente 3a bis 3n der Fig. 1, wobei jedoch die laterale Auflösung erheblich besser ist. Vergleicht man indes aktive Flächen, so ergibt sich beim Fein-Array der Fig. 2 sogar eine um etwa +4 dB höhere Echoantwort als beim herkömmlichen Array der Fig. 1.

Bei einem Ausführungsbeispiel der Praxis umfaßt das Ultraschall-Array der Fig. 2 bei einer Ultraschallfrequenz von f = 2,25 MHz insgesamt 256 Einzelschwinger 11a, 12a, bis 11n, 12n. Die Breite b eines jeden Einzelschwingers beträgt etwa 0,26 mm. Die Höhe der Elemente beträgt etwa h = 0,7 mm und die Länge ungefähr I = 11 mm. Bei einer Spaltbreite in der Größenordnung von s = 0,05 bis 0,06 mm ergibt sich damit bei Zusammenfassung von insgesamt zwei Wandlerelementen zu einer Gruppe eine Gesamtgruppenbreite von etwa 0,57 mm, wobei die einzelnen Gruppen untereinander entlang dem Wandlerkamm 9 durch Spaltbreiten wiederum in der Größenordnung s = 0,05 bis 0,06 mm abgetrennt sind.

Die Feinunterteilung des Wandlerkammes gemäß dem Array der Fig. 2 kann z.B. in Sägetechnik mittels Gattersäge oder auch mittels Schneidstrahl, z.B. Laser- oder Ultraschall-Schneidstrahl, vorgenommen werden. Ebensogut ist auch Anwendung von Stapeltechnik dünner Folien, LSI-Fotoresist- und Ätztechnik oder auch Sintertechnik in Stäbchenform möglich.

Die Wandlerelemente 11a, 12a bis 11n, 12n, können die in Fig. 2 dargestellte Stäbchenform mit über die Wandlerlänge 1 und Wandlerhöhe h gleichbleibender Breite b haben. Ebensogut sind jedoch auch andere geometrische Formgebungen möglich, z.B. Trapezform nach Höhe h und/oder Länge 1, wobei jedoch die Breite b zumindest an einer Stelle des einzelnen Wandlerelementes den Bedingungen der Erfindung genügen sollte. Ausführungsbeispiele dieser Art zeigen die Fig. 3 bis 5 mit Wandlerelementformen 21a bis 21n oder 22a bis 22n.

Das Ausführungsbeispiel der Fig. 2 arbeitet mit zwei Einzelelementen 11a, 12a etc. bis 11n, 12n pro Gruppe. Selbstverständlich können auch mehr als nur zwei, z.B. drei, vier oder mehr Einzelelemente, zu einer Wandlergruppe zusammengeschaltet werden. Die Einzelelemente können dann noch feiner unterteilt sein, so daß sich beispielsweise eine Breitenbedingung erheblich kleiner als $\lambda/4$ und damit sehr viel kleiner als $\lambda/2$ ergibt. Die Gesamtbreite jeder Wandlergruppe kann dann wieder etwa $\lambda/2$ sein. Ein solches Ultraschall-Array eignet sich dann insbesondere wieder zur Anwendung für Sektor-Abtastung. Ebensogut kann jedoch auch die Gesamtbreite einer Wandlergruppe größer

als $\lambda/2$ sein. Derartige Ausbildungsformen finden Einsatz z.B. bei Linear-Arrays.

Entsprechendes gilt auch für die Ausführungsform der Fig. 6, die einen Schallkopf 23 insbesondere für Ultraschall-Compound-Scan zeigt. Die Ultraschallwandlerelemente 24a bis 24n bilden wieder eine jetzt jedoch runde kammförmige Abstrahl- bzw. Empfangsfläche mit Gruppenschaltung 25. 26 ist dabei das Anschlußkabel für den Schallkopf. Die Stäbchenausführung der Fig. 6 kann bei Bedarf durch eine Mosaik- bzw. Matrix-Ausführung, z.B. durch Bildung zusätzlicher Querspalten, ersetzt werden. Dasselbe gilt selbstverständlich auch für die Array-Kamm-Ausführungen der Fig. 1 bis 5.

## Patentansprüche

1. Ultraschallkopf, bestehend aus einer vorgebbaren Zahl von in bestimmter Flächenformation aneinandergereihten Wandlerelementen, deren Breite wenigstens stellenweise unterhalb $\lambda/4$, mit $\lambda$ als Wellenlänge der abgestrahlten bzw. empfangenen Ultraschallwellen, liegt und kleiner ist als ihre Höhe und von denen einzelne benachbarte Wandlerelemente in Gruppen von wenigstens zwei Einzelelementen elektrisch parallel zusammengeschaltet sind, dadurch gekennzeichnet, daß die Gesamtzahl aller Wandlerelemente (11a, 12a bis 11n, 12n; 21a bis 21n; 22a bis 22n; 24a bis 24n) in eine Vielzahl von Gruppen (13) unterteilt ist, in welchen jeweils eine solche Zahl von benachbarten Wandlerelementen elektrisch parallel zusammenkontaktiert sind, daß die Summen-Abstrahl- bzw. Empfangsfläche der Gruppe einschließlich der Zwischenräume (20) wenigstens so groß ist wie die Aktivfläche eines Wandlerelementes (3a bis 3n) einer herkömmlichen Wandleranordnung (1), dessen Wandlerelementbreite (B) im Bereich von $\lambda/2$ oder allenfalls geringfügig darunter liegt und dessen Wandlerelementhöhe (h) größer als die Wandlerelementbreite (B) ist und in welchen die Höhe (h) der Wandlerelemente dieselbe ist wie die Wandlerelementhöhe (h) der herkömmlichen Wandlerelementes.

2. Ultraschallkopf nach Anspruch 1, dadurch gekennzeichnet, daß die Zwischenräume (20) Spaltbreiten (s) in der Größenordnung von maximal 1/5 der jeweiligen Wandlerelementbreite (b) aufweisen.

3. Ultraschallkopf nach Anspruch 1 oder 2, dadurch gekennzeichnet, daß jeder Gruppenkontaktierung (14; 16; 25) eine eigene Signalleitung (18, 19) zur Ansteuerung der Gruppe (13) zugeordnet ist.

4. Ultraschallkopf nach einem der Ansprüche 1 bis 3, gekennzeichnet durch stabförmige Wandlerelemente (11a, 12a bis 11n, 12n) mit über Elementlänge (1) und Elementhöhe (h) gleichbleibender Breite $b < \lambda/4$ (Figur 2).

5. Ultraschallkopf nach einem der Ansprüche 1 bis 3, gekennzeichnet durch Wandlerelemente (21a bis 21n) mit über die Elementhöhe (h) gleichbleibender, jedoch über die Elementlänge (1) von einem Niedrigwert kleiner $\lambda/4$ zu einem Höchstwert, vorzugsweise trapezförmig, zunehmender Breite (b), wobei der Höchstwert im Bereich $\lambda/2$ oder darüber liegen kann (Figuren 3, 4).

6. Ultraschallkopf nach einem der Ansprüche 1 bis 3, gekennzeichnet durch Wandlerelemente (22a bis 22n) mit über die Elementlänge (l) gleichbleibender, jedoch über die Elementhöhe (h) von einem Niedrigwert kleiner $\lambda/4$ zu einem Höchstwert, vorzugsweise trapezförmig, zunehmender Breite (b), wobei der Höchstwert im Bereich $\lambda/2$ oder darüber liegen kann (Figur 5).

7. Ultraschallkopf nach einem der Ansprüche 1 bis 6, gekennzeichnet durch eine Matrix- oder Mosaikanordnung der Wandlerelemente, insbesondere aufgrund von Längs- und Querspalten.

8. Ultraschallkopf nach einem der Ansprüche 1 bis 3, gekennzeichnet durch die Anordnung stab- oder mosaikförmiger Wandlerelemente (24a bis 24n) zu einer runden, insbesondere kreisförmigen, Platte (Figur 6).

## Revendications

1. Tête ultrasonore, constituée par un nombre prédéterminable d'éléments transducteurs disposés les uns à côté des autres suivant une formation superficielle déterminée, dont la largeur est, au moins par endroit, inférieure à $\lambda/4$, $\lambda$ étant la longueur d'ondes ultrasonores émises ou reçues, et est plus faible que la hauteur, des éléments transducteurs individuels voisins étant réunis, en parallèle du point de vue électrique, en groupes d'au moins deux éléments individuels, caractérisée par le fait que le nombre total de tous les éléments transducteurs (11a, 12a à 11n, 12n; 21a à 21n; 22a à 22n; 24a à 24n) est subdivisé en plusieurs groupes (13) qui comportent respectivement un nombre d'éléments transducteurs voisins réunis en parallèle du point de vue électrique tel que la somme des surfaces de rayonnement ou de réception du groupe, y compris les espaces intermédiaires (20), est au moins aussi importante que la surface active d'un élément transducteur (3a à 3n) d'un dispositif transducteur classique (1), dont la largeur (B) des éléments transducteurs se trouve dans la gamme de $\lambda/4$ ou, à la rigueur, en est légèrement inférieure, et dont la hauteur (h) des éléments transducteurs est plus importante que la largeur (B) des éléments transducteurs, et dans lesquels la hauteur (h) des éléments transducteurs est la même que la hauteur (h) des éléments transducteurs du dispositif transducteur classique.

2. Tête ultrasonore suivant la revendication 1, caractérisée par le fait que les espaces intermédiaires (20) possèdent des largeurs d'intervalles (s) de l'ordre de grandeur d'au maximum 1/5 de la largeur respective (b) de l'élément transducteur.

3. Tête ultrasonore suivant la revendication 1 ou 2, caractérisée par le fait qu'à chaque élément de contact (14; 16; 25) des groupes est associée une ligne de signaux (18, 19) pour la commande du groupe (13).

4. Tête ultrasonore selon l'une des revendications 1 à 3, caractérisée par le fait qu'elle comporte des éléments transducteurs (11a, 12a à 11n, 12n) en forme de barreaux qui possèdent une largeur $b < \lambda/4$ constante sur la longueur (l) de l'élément et sur la hauteur (h) de l'élément (figure 2).

5. Tête ultrasonore suivant l'une des revendications 1 à 3, caractérisée par le fait qu'elle comporte des éléments transducteurs (21a à 21n) qui possèdent une largeur (b) qui reste constante sur la hauteur (h) de l'élément mais, de préférence, augmente en forme trapézoïdale, d'une valeur faible inférieure à $\lambda/4$ jusqu'à une valeur maximale, sur la longueur (l) de l'élément, la valeur maximale pouvant se trouver dans la gamme de $\lambda/2$ ou être supérieure à celle-ci (figures 3 et 4).

6. Tête ultrasonore suivant l'une des revendications 1 à 3, caractérisée par le fait qu'elle comporte des éléments transducteurs (22a à 22n) qui possèdent une largeur (b) qui est constante sur la longueur (l) de l'élément mais, de préférence, augmente en forme trapézoïdale, d'une valeur faible inférieure à $\lambda/4$ jusqu'à une valeur maximale, sur la hauteur (h) de l'élément, la valeur maximale pouvant se trouver dans la gamme $\lambda/2$ ou être supérieure à celle-ci (figure 5).

7. Tête ultrasonore suivant l'une des revendications 1 à 6, caractérisée par un agencement en forme de matrice ou de mosaïque des éléments transducteurs, notamment sur la base d'intervalles longitudinaux et transversaux.

8. Tête ultrasonore suivant l'une des revendications 1 à 3, caractérisée par l'agencement d'éléments transducteurs, en forme de barreaux ou de tiges (24a à 24n), mis sous la forme d'une plaque ronde, notamment circulaire (figure 6).

## Claims

1. An ultrasonic head comprising a given number of transducer elements of a width below $\lambda/4$ at least at certain portions thereof, $\lambda$ being the wavelength of the emitted or received ultrasonic waves, as the case may be, the element width being smaller than their height, and the elements being joined together in a specific areal formation with individual adjacent transducer elements electrically interconnected in parallel in groups of at least two individual elements, characterised in that the overall number of all transducer elements (11a, 12a to 11n, 12n; 21a to 21n; 22a to 22n; 24a to 24n) is divided into a plurality of groups (13) in which such a number of adjacent transducer elements are electrically interconnected in parallel that the sum-emission-surface or sum-receiving-surface, as the case may be, of the group including the interspaces (20) is at least as large as the active surface of one transducer element (3a to 3n) of a conventional transducer arrangement (1) whose transducer element width (B) lies in the region of or slightly below $\lambda/2$ and whose transducer element height (h) is larger than the transducer element width (B), the height (h) of the individual transducer elements being the same as the transducer element height (h) of the conventional transducer element.

2. An ultrasonic head as claimed in Claim 1, characterised in that the interspaces (20) have gap widths (s) in the order of a maximum of 1/5 of the relevant transducer element width (b).

3. An ultrasonic head as claimed in Claim 1 or 2, characterised in that to each group interconnection arrangement (14; 16; 25) there is assigned a separate signal line (18, 19) for controlling the group (13).

4. An ultrasonic head as claimed in one of Claims 1 to 3, characterised by rod-shaped transducer elements (11a, 12a to 11n, 12n) having a width $b < \lambda/4$ which is constant with respect to element length (l) and element height (h) (Figure 2).

5. An ultrasonic head as claimed in one of Claims 1 to 3, characterised by transducer elements (21a to 21n) having a width (b) which is constant with respect to the element height (h) but increases, preferably in trapezoidal fashion, with respect to the element length (l) from a low value smaller than $\lambda/4$ to a maximum value which value is in the region of or above $\lambda/2$ (Figures 3, 4).

6. An ultrasonic head as claimed in one of Claims 1 to 3, characterised by transducer elements (22a to 22n) having a width (b) which is constant with respect to the element length (l), but increases, preferably in trapezoidal fashion, with respect to the element height (h) from a low value smaller than $\lambda/4$ to a maximum value which value is in the region of or above $\lambda/2$ (Figure 5).

7. An ultrasonic head as claimed in one of Claims 1 to 6, characterised by a matrix arrangement or mosaic arrangement of the transducer elements, in particular as a result of longitudinal gaps and transverse gaps.

8. An ultrasonic head as claimed in one of Claims 1 to 3, characterised by the arrangement of rod-shaped or mosaic-shaped transducer elements (24a to 24n) so as to form a roundish, in particular circular plate (Figure 6).

FIG 1

FIG 2

FIG 3

FIG 4

FIG 5

FIG 6